# EUROPEAN PATENT SPECIFICATION

(11) **EP 4 438 099 B1**
(45) Date of publication and mention of the grant of the patent: **08.04.2026**
(21) Application number: 22898265.8
(22) Date of filing: 14.10.2022
(51) Int. Cl.: A61M 25/09, A61M 25/098, A61M 25/01

(54) **GUIDE WIRE**
FÜHRUNGSDRAHT
FIL GUIDE

(30) Priority: 26.11.2021 JP 2021192234
(43) Date of publication of application: 02.10.2024
(73) Proprietor: ASAHI INTECC CO., LTD., Seto-shi, Aichi, 489-0071 (JP)
(72) Inventor: PHAM Hoang Viet, Seto-shi, Aichi 489-0071 (JP); ENDOU, Natsuko, Seto-shi, Aichi 489-0071 (JP); IKUTA, Masahiro, Seto-shi, Aichi 489-0071 (JP)
(74) Representative: Winter, Brandl - Partnerschaft mbB
(86) International application number: PCT/JP2022/038325
(87) International publication number: WO 2023/095481

(56) References cited:
- WO-A1-2020/219457
- WO-A1-2020/219457
- WO-A1-91/00051
- JP-A- 2007 044 388
- JP-A- 2014 161 705
- US-A- 5 488 959
- US-A- 5 488 959

## Description

### TECHNICAL FIELD

The present invention relates to a guide wire.

### BACKGROUND ART

When treating stenosis that occurs in blood vessels such as coronary arteries surrounding a heart, or when treating a site where a blood vessel is completely occluded (e.g., chronic total occlusion: CTO, etc.) due to a progression of calcification, a guide wire for guiding treatment instruments such as a balloon catheter is inserted into the blood vessel prior to the treatment instruments.

In a guide wire of US4634042A, a head is formed by melting only a distal end of a core wire made up of stainless steel. The core wire and a distal end of a wire coil are connected by the head. US 5 488 959 A relates to guidewires for use with medical catheters and to a welding procedure useful in manufacturing guidewires and other devices incorporating welding techniques. WO 2020/219457 A1 is directed to advancements in pressure guidewire technology. WO 91/00051 A1 relates to medical devices formed from elongated wires and coils used as guidewires, e.g., for navigating narrow passageways of a body.

### SUMMARY OF INVENTION

### TECHNICAL PROBLEM

However, in a case of the guide wire of US4634042A, since a distal end thereof is composed of only stainless steel, which is a material through which X-rays are easily transmitted, visibility of a distal end portion of the guide wire during X-ray irradiation is not so good.

The present disclosure has been made based on the above circumstances, and an object of the present disclosure is to provide a guide wire that is capable of improving the visibility of the distal end portion thereof during X-ray irradiation.

### SOLUTION TO PROBLEM

This object is solved by the subject matter of the independent claim. Further aspects are disclosed in the dependent claims. In order to solve the above-described problem, a guide wire according to an aspect of the disclosed embodiments includes: a core shaft; a coil made up of a strand that is spirally wound to cover an outer periphery of the core shaft; and a distal end joint part that joins a distal end of the core shaft and a distal end of the coil. The distal end joint part includes a dispersion portion in which a first material that is radiopaque is dispersed. The distal end joint part encloses a distal end portion of the coil, and at the distal end portion of the coil, a diameter of the strand of the coil decreases toward a distal end of the distal end portion.

The distal end joint part may include a high concentration region in which a concentration of the first material is relatively high.

The high concentration region may be located on a rear end side of the distal end joint part.

The core shaft may be made up of a second material different from the first material, the coil may be made up of the first material, and the distal end joint part may be formed by melting and joining a distal end portion of the core shaft and a distal end portion of the coil.

The strand in the distal end joint part may have a convex portion.

### EFFECT OF THE INVENTION

According to the disclosed embodiments, it is possible to provide a guide wire capable of improving visibility of a distal end portion thereof during X-ray irradiation.

### BRIEF DESCRIPTION OF DRAWINGS

FIG. 1 is a partial cross-sectional schematic view of a guide wire according to an embodiment.
FIG. 2 is an enlarged partial cross-sectional view of a vicinity of a distal end joint part of the guide wire in FIG. 1.
FIG. 3 is an image of a longitudinal cross-section of the distal end joint part.
FIG. 4 is an element mapping image of a longitudinal cross-section of the distal end joint part.
FIG. 5 is an explanatory view of a distal end portion and a high concentration region of the distal end joint part.
FIG. 6 is an element mapping image of a longitudinal cross-section of the distal end joint part, which is an element mapping image with strands enlarged.

### EMBODIMENTS OF THE INVENTION

Hereinafter, an embodiment of the present disclosure will be explained with reference to the drawings, but the disclosed embodiments are not limited only to the embodiments described in the drawings. Note that, in the present disclosure, a distal end means an end portion of the guide wire at which a distal end joint part is located, and a rear end means an end portion on a side opposite to the distal end.

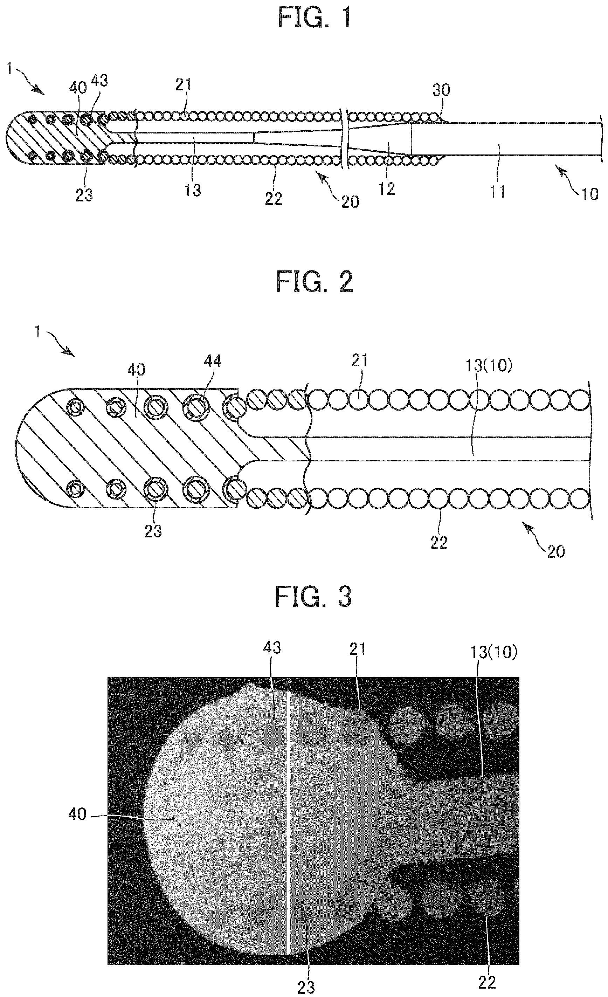

FIG. 1 is a partial cross-sectional schematic view of a guide wire 1 according to an embodiment. FIG. 2 is an enlarged partial cross-sectional view of a vicinity of a distal end joint part of the guide wire 1. FIG. 1 illustrates only a part of a distal end side of the guide wire 1.

The guide wire 1 includes a core shaft 10, a coil 20, a proximal end joint part 30 and a distal end joint part 40.

The core shaft 10 includes a core shaft main body 11, a tapered portion 12, and a distal end portion 13. The tapered portion 12 extends continuously from the core shaft main body 11 while decreasing in diameter toward the distal end. The distal end portion 13 extends continuously from the tapered portion 12 toward the distal end. Although not described in FIG. 1, the core shaft main body 11 extends toward the rear end side, and a user performs a rotation operation or the like of the guide wire 1 at an end portion on the rear end side of the core shaft main body 11.

Examples of the material constituting the core shaft 10 include stainless steels such as SUS302, SUS304, and SUS316.

As illustrated in FIG. 1, the coil 20 is provided to cover an outer periphery of the distal end of the core shaft main body 11, the tapered portion 12, and the distal end portion 13. The coil 20 is formed in a hollow cylindrical shape by spirally winding a strand 21 made up of, for example, tungsten around the core shaft 10.

The coil 20 has a rear end portion 22 and a distal end portion 23. At the rear end portion 22, the strand 21 is wound to be in contact with each other in the axial direction. At the distal end portion 23, the strand 21 is wound to have a gap in the axial direction. The distal end portion 23 has an outer diameter smaller than that of the rear end portion 22. The distal end portion 23 is included in the distal end joint part 40. The material constituting the coil 20 is, for example, tungsten that is radiopaque. Tungsten constituting the coil 20 is used as a first material that is radiopaque. The stainless steel constituting the core shaft 10 is used as a second material different from the first material.

A proximal end of the coil 20 is joined to the core shaft 10 by the proximal end joint part 30. Examples of the material constituting the proximal end joint part 30 include a brazing metal such as an Sn-Pb alloy, a Pb-Ag alloy, an Sn-Ag alloy, or an Au-Sn alloy, and an adhesive such as an epoxy-based resin.

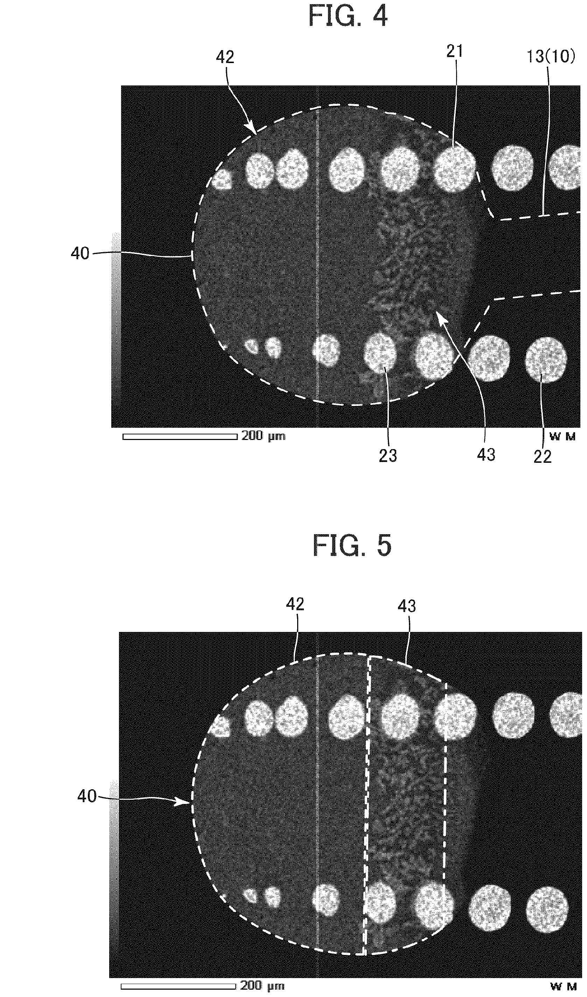

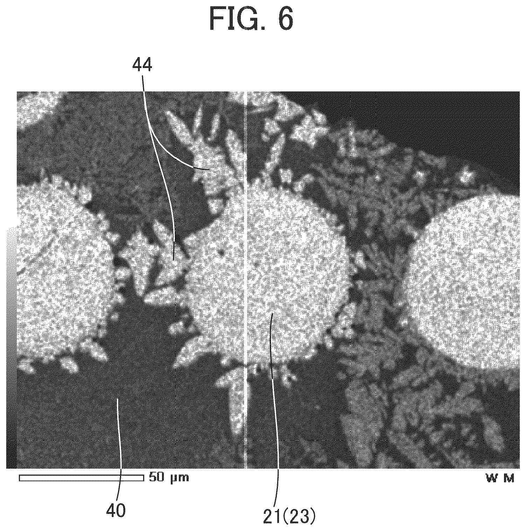

Next, the distal end joint part 40 will be explained. FIG. 3 is an image of a longitudinal cross-section of the distal end joint part 40. FIG. 4 is an element mapping image of a longitudinal cross-section of the distal end joint part 40. FIG. 5 is an explanatory view of a dispersion portion 42 and a high concentration region 43 of the distal end joint part 40. FIG. 6 is an element mapping image of a longitudinal cross-section of the distal end joint part 40, which is an element mapping image with the enlarged strand 21. In FIGS. 4 and 5, outer shapes of the distal end joint part 40 and the distal end portion 13 of the core shaft 10 are indicated by white dotted lines. FIGS. 4 to 6 are element mapping images of a longitudinal cross-section of the distal end joint part 40 acquired by using energy dispersive X-ray spectroscopy (EDX).

The distal end joint part 40 joins the distal end portion 13 of the core shaft 10 with the distal end portion 23 of the coil 20. The distal end joint part 40 encloses the distal end portion 23 of the coil 20. A diameter of the strand 21 of the distal end portion 23 decreases toward the distal end. The distal end of the distal end portion 23 is located near the distal end of the distal end joint part 40. The distal end joint part 40 is formed by melting a portion of the core shaft 10 originally located on the distal end side with respect to the distal end portion 13 and a part of the strand 21 of the distal end portion 23 by arc welding such as plasma welding.

As illustrated in FIG. 4, in the distal end joint part 40, a black portion represents a material other than the first material such as stainless steel, and a gray or white portion represents the first material such as tungsten. The distal end joint part 40 includes a dispersion portion 42 in which the first material having radiopacity exists in a manner substantially uniformly dispersed in a certain range, and a high concentration region 43 in which a concentration of the first material is higher than that in the dispersion portion 42. In detail, in the distal end joint part 40 of FIG. 5, a portion of a region surrounded by a dotted line except for the strands 21 is the dispersion portion 42, and a portion of a region surrounded by an alternate long and short dash line except for the strands 21 is the high concentration region 43. The high concentration region 43 exists on a rear end side of the distal end joint part 40.

The dispersion portion 42 is a portion in which the first material (tungsten) having radiopacity exists in a manner substantially uniformly dispersed in a certain range. In the dispersion portion 42 of the present embodiment, tungsten exists in a manner substantially uniformly dispersed in a certain range. In the present embodiment, the dispersion portion 42 is, for example, a portion where the molar ratio (%) of tungsten per unit area (1µm²) in the distal end joint part 40 is greater than 0% and less than 13.3%. The concentration of the first material in the dispersion portion 42 is relatively low compared to the high concentration region 43. In the present embodiment, the dispersion portion 42 exists to spread over the entire distal end side in the distal end joint part 40. As a result, the entire distal end side of the distal end joint part 40 can be visually recognized under radioscopy.

The high concentration region 43 is a portion in which the concentration of the first material having radiopacity is relatively high compared to the dispersion portion 42. In the present embodiment, the high concentration region 43 is, for example, a portion where the molar ratio (%) of tungsten per unit area (1µm²) is 13.3% or more. The high concentration region 43 may be provided in any portion of the distal end joint part 40. When the high concentration region 43 exists at the distal end of the distal end joint part 40, the visibility of the distal end of the distal end joint part 40 under radioscopy is improved. When the high concentration region 43 exists at the rear end of the distal end joint part 40, visibility of a boundary portion between the distal end joint part 40 and the core shaft 10 under radioscopy is improved.

As illustrated in FIG. 6, a dendritic phase 44 made up of tungsten and extending outward in a dendritic shape from the strand 21 exists around the strand 21. The dendritic phase 44 exists to spread radially on the outer periphery of the strand 21. Namely, the strand 21 in the distal end joint part 40 has a convex portion on the outer periphery thereof. As described above, in the distal end joint part 40, a part of the strand 21 made up of tungsten is melted by arc welding at the time of forming the distal end joint part 40, and the melted tungsten is distributed in the distal end joint part 40 as the dispersion portion 42 and the high concentration region 43 and around the strand 21 as the dendritic phase 44.

Next, a usage mode of the guide wire 1 will be explained. The guide wire 1 is inserted into a blood vessel in a femoral region from the distal end joint part 40 and advanced to a coronary artery along the blood vessel. Next, after passing through a treated site such as a constricted part of a blood vessel or a false lumen in the vicinity of CTO, a treatment instrument such as a balloon catheter or a stent is conveyed along the guide wire 1, and various treatments are performed at the treated site. After the above-described treatments are completed, the guide wire 1 is made retrograde relative to the blood vessel and drawn out from a body, whereby a series of procedures is completed. A position of the distal end portion of the guide wire 1 in the blood vessel is confirmed by a radioscopic image.

The above-described guide wire 1 includes the core shaft 10, the coil 20 made up of a strand that is spirally wound to cover the outer periphery of the core shaft 10, and the distal end joint part 40 that joins the distal end of the core shaft 10 and the distal end portion 23 of the coil 20. The distal end joint part 40 includes the dispersion portion 42 in which tungsten, which is radiopaque, is dispersed. This makes it possible to improve the visibility of the distal end joint part 40, which is the distal end portion of the guide wire 1, during X-ray irradiation.

The distal end joint part 40 has the high concentration region 43 in which a concentration of tungsten is relatively high. This makes it possible to further improve the visibility of the distal end joint part 40, which is the distal end portion of the guide wire 1, during X-ray irradiation.

The high concentration region 43 is located on the rear end side of the distal end joint part 40. As a result, it is possible to further improve the visibility of the rear end portion (the distal end of the distal end portion 13) of the distal end joint part 40, which is the distal end portion of the guide wire 1, during X-ray irradiation.

The core shaft 10 is made up of stainless steel different from tungsten, the coil 20 is made up of tungsten, and the distal end joint part 40 is formed by melting and joining the distal end portion 13 of the core shaft 10 and the distal end portion 23 of the coil 20. In this way, the distal end joint part 40 is integrally formed by melting the core shaft 10 and the coil 20, whereby a joint strength between the core shaft 10 and the coil 20 can be further improved.

Since the strand 21 in the distal end joint part 40 has a convex portion, the joint strength between the core shaft 10 and the coil 20 can be further improved.

Note that the disclosed embodiments are not limited to the configuration of the above-mentioned embodiment, but is defined by the scope of claims, and is intended to include all modifications within the meaning and scope of equivalents to the scope of claims.

For example, in the above-described embodiment, the coil 20 is constituted by one strand 21, but may be constituted by a plurality of strands. The dispersion portion 42 is constituted of tungsten as a first material that is radiopaque and stainless steel as a second material through which X-rays are easily transmitted. However, other materials through which X-rays are easily transmitted and materials that is radiopaque may be used as long as it is possible to form a distal end joint part having a dispersion portion in which the first material having radiopacity exists in a manner substantially uniformly dispersed in a certain range of the second material through which X-rays are easily transmitted.

### DESCRIPTION OF REFERENCE NUMERALS

- 1: Guide wire
- 10: Core shaft
- 20: Coil
- 23: Distal end portion
- 40: Distal end joint part
- 42: Dispersion portion
- 43: High concentration region
- 44: Dendritic phase

## Claims

1. A guide wire (1) comprising:
a core shaft (10);
a coil (20) made up of a strand that is spirally wound to cover an outer periphery of the core shaft (10); and
a distal end joint part (40) that joins a distal end of the core shaft (10) and a distal end of the coil (20), wherein
the distal end joint part (40) has a dispersion portion (42) in which a first material that is radiopaque is dispersed, **characterized in that**
the distal end joint part (40) encloses a distal end portion (23) of the coil (20), and
at the distal end portion (23) of the coil (20), a diameter of the strand (21) of the coil (20) decreases toward a distal end of the distal end portion (23).

2. The guide wire (1) according to claim 1, wherein the distal end joint part (40) includes a high concentration region (43) in which a concentration of the first material is higher than a concentration of the first material in the dispersion portion (42).

3. The guide wire (1) according to claim 2, wherein the high concentration region (43) is located on a rear end side of the distal end joint part (40).

4. The guide wire (1) according to any one of claims 1 to 3, wherein
the core shaft (10) is made up of a second material different from the first material,
the coil (20) is made up of the first material, and
the distal end joint part (40) is formed by melting and joining a distal end portion (13) of the core shaft (10) and a distal end portion (23) of the coil (20).

5. The guide wire (1) according to any one of claims 1 to 4, wherein the strand in the distal end joint part (40) has a convex portion.

6. The guide wire (1) according to any one of claims 1 to 5, wherein an outer diameter of a distal end portion (23) of the coil (20) at the distal end joint part (40) is smaller than an outer diameter of a rear end portion (22) of the coil (20).

7. The guide wire (1) according to claim 5, wherein the convex portion spreads radially outward from an outer periphery of the strand (21) of the coil (20).

## Patentansprüche

1. Führungsdraht (1), mit
einem Kernschaft (10),
einer Spule (20), die aus einem Strang besteht, der spiralförmig gewickelt ist, um einen Außenumfang des Kernschafts (10) zu bedecken, und
einem distalen Endverbindungsteil (40), der ein distales Ende des Kernschafts (10) und ein distales Ende der Spule (20) verbindet, wobei
der distale Endverbindungsteil (40) einen Dispersionsabschnitt (42) aufweist, in dem ein erstes röntgendichtes Material dispergiert ist, **dadurch gekennzeichnet, dass**
der distale Endverbindungsteil (40) einen distalen Endabschnitt (23) der Spule (20) einschließt und
sich am distalen Endabschnitt (23) der Spule (20) der Durchmesser des Strangs (21) der Spule (20) zum distalen Ende des distalen Endabschnitts (23) hin verringert.

2. Führungsdraht (1) gemäß Anspruch 1, wobei der distale Endverbindungsteil (40) einen Hochkonzentrationsbereich (43) aufweist, in dem die Konzentration des ersten Materials höher ist als die Konzentration des ersten Materials im Dispersionsabschnitt (42).

3. Führungsdraht (1) gemäß Anspruch 2, wobei sich der Hochkonzentrationsbereich (43) an einer hinteren Endseite des distalen Endverbindungsteils (40) befindet.

4. Führungsdraht (1) gemäß einem der Ansprüche 1 bis 3, wobei
der Kernschaft (10) aus einem zweiten Material besteht, das sich vom ersten Material unterscheidet,
die Spule (20) aus dem ersten Material besteht und
der distale Endverbindungsteil (40) durch Schmelzen und Verbinden eines distalen Endabschnitts (13) des Kernschafts (10) und eines distalen Endabschnitts (23) der Spule (20) gebildet ist.

5. Führungsdraht (1) gemäß einem der Ansprüche 1 bis 4, wobei der Strang im distalen Endverbindungsteil (40) einen konvexen Abschnitt aufweist.

6. Führungsdraht (1) gemäß einem der Ansprüche 1 bis 5, wobei ein Außendurchmesser eines distalen Endabschnitts (23) der Spule (20) am distalen Endverbindungsteil (40) kleiner ist als ein Außendurchmesser eines hinteren Endabschnitts (22) der Spule (20).

7. Führungsdraht (1) gemäß Anspruch 5, wobei sich der konvexe Abschnitt radial nach außen von einem Außenumfang des Strangs (21) der Spule (20) erstreckt.

## Revendications

1. Fil guide (1) comprenant :
une âme centrale (10) ;
une bobine (20) constituée d'un brin enroulé en hélice pour recouvrir une périphérie externe de l'âme centrale (10) ; et
une partie de jonction d'extrémité distale (40) qui relie une extrémité distale de l'âme centrale (10) et une extrémité distale de la bobine (20), dans lequel
la partie de jonction d'extrémité distale (40) présente une partie de dispersion (42) dans laquelle est dispersé un premier matériau radio-opaque, **caractérisé en ce que**
la partie de jonction d'extrémité distale (40) renferme une partie d'extrémité distale (23) de la bobine (20), et
à l'extrémité distale (23) de la bobine (20), le diamètre du brin (21) de la bobine (20) diminue vers l'extrémité distale de la partie d'extrémité distale (23).

2. Fil guide (1) selon la revendication 1, dans lequel la partie de jonction d'extrémité distale (40) inclut une région à forte concentration (43) dans laquelle la concentration du premier matériau est supérieure à la concentration du premier matériau dans la partie de dispersion (42).

3. Fil guide (1) selon la revendication 2, dans lequel la région à forte concentration (43) est située sur le côté arrière de la partie de jonction distale (40).

4. Fil guide (1) selon l'une quelconque des revendications 1 à 3, dans lequel
l'âme centrale (10) est constituée d'un second matériau différent du premier matériau,
la bobine (20) est constituée du premier matériau, et
la partie de jonction d'extrémité distale (40) est formée en faisant fondre et en joignant une partie d'extrémité distale (13) de l'âme centrale (10) et une partie d'extrémité distale (23) de la bobine (20).

5. Fil guide (1) selon l'une quelconque des revendications 1 à 4, dans lequel le brin de la partie de jonction d'extrémité distale (40) présente une portion convexe.

6. Fil guide (1) selon l'une quelconque des revendications 1 à 5, dans lequel un diamètre extérieur d'une partie d'extrémité distale (23) de la bobine (20) au niveau de la partie de jonction d'extrémité distale (40) est plus petit qu'un diamètre extérieur d'une partie d'extrémité arrière (22) de la bobine (20).

7. Fil guide (1) selon la revendication 5, dans lequel la partie convexe s'étend radialement vers l'extérieur à partir d'une périphérie extérieure du brin (21) de la bobine (20).
